# EUROPEAN PATENT SPECIFICATION

(11) **EP 1 910 830 B1**
(45) Date of publication and mention of the grant of the patent: **28.10.2009**
(21) Application number: 06766060.5
(22) Date of filing: 10.07.2006
(51) Int. Cl.: G01N 33/543, G01N 33/544, G01N 33/548

(54) **SUBSTRATE MATERIAL FOR ANALYZING FLUIDS**
SUBSTRATMATERIAL ZUR ANALYSE VON FLÜSSIGKEITEN
MATÉRIAU SUBSTRAT POUR ANALYSE DE FLUIDES

(30) Priority: 21.07.2005 EP 05106687
(43) Date of publication of application: 16.04.2008
(73) Proprietor: Koninklijke Philips Electronics N.V., 5621 BA Eindhoven (NL)
(72) Inventor: KURT, Ralph, Philips IP & Standards GmbH, 52066 Aachen (DE); STAPERT, Hendrik R., Philips IP& Standards GmbH, 52066 Aachen (DE); JOHNSON, Mark Thomas, Philips IP & Standards GmbH, 52066 Aachen (DE); BROER, Dirk Jan, Philips IP & Standards GmbH, 52066 Aachen (DE); PENTERMAN, Roel, Philips IP & Standards GmbH, 52066 Aachen (DE); PEETERS, Emiel, Philips IP &Standards GmbH, 52066 Aachen (DE)
(74) Representative: Schouten, Marcus Maria
(86) International application number: PCT/IB2006/052327
(87) International publication number: WO 2007/010432

(56) References cited:
- EP-A- 0 167 298
- WO-A-03/025574
- US-A- 4 708 931
- US-A1- 2002 160 538
- US-A1- 2004 023 289
- STIMPSON D I ET AL: "PARALLEL PRODUCTION OF OLIGONUCLEOTIDE ARRAYS USING MEMBRANES AND REAGENT JET PRINTING" BIOTECHNIQUES, INFORMA LIFE SCIENCES PUBLISHING, WESTBOROUGH, MA, US, vol. 25, no. 5, November 1998 (1998-11), pages 886-890, XP001109712 ISSN: 0736-6205

## Description

The present invention is directed to the field of devices for the detection of one or more analytes in a fluid sample, especially to the field of devices for the detection of biomolecules in aqueous solution.

The present invention is directed to the detection of analytes in fluids, especially to the detection of biomolecules in an aqueous solution. The detection usually occurs in that way, that the fluid to be analyzed is provided on a substrate material, which contains binding substances for the analytes which are subject of the detection. Such a capture probe may be a corresponding DNA-strand in case the analyte is also a DNA strand. The analytes in the fluid, which are usually equipped with a label, preferably an optical fluorescence label, will then be captured by the binding substance (in case of two complementary DNA strands this process is called hybridization) and remain there even after the fluid is removed. The analyte may then be detected.

Such a detection device is disclosed in the US 6,849,408 B2. However, in devices such as disclosed in the US 6, 849, 408 B2 or other devices of the prior art, a substrate material was used, which consisted out of through-going channels. This has the drawback that there has to be a certain amount of analyte in the fluid, since the fluid is not spread over the whole substrate material during detection.

Furthermore, substrate materials such as large-pore nitrocellulose are known in the field. These materials allow a lateral distribution of fluid within the substrate material, however, this distribution cannot be controlled.

US 4,708,931 describes a laminated rod having alternating detection and spacer layers for binding assays that is used in a microassay card application. Stimpson et al (BioTechniques, 25:886-890, 1998) describe a parallel production of oligonucleotide arrays using membranes and reagent jet printing, wherein lines of reagent are printed in parallel on a membrane support. US 2002/0160538 describes an assay device for determining the presence or absence of an analyte in a test sample, wherein said device includes a reaction medium with at least one reaction zone and at least one control zone. EP 0 167 298 describes a method an an apparatus for clinical, chemical and biological testing, wherein a macro-solid phase absorbent test material is utilized. WO 03/025575 describes an embossed test strip system. US 2004/0023289 describes a method and a device for obtaining and detecting immunologically active substances from the gas phase utilizing a carrier matrix.

It is therefore an object of the present invention to provide a device, which allows a quicker detection with a minute amount of analyte that needs to be present in the fluid.

This object is solved by a device according to claim 1 of the present invention. Accordingly, a device for analyzing one or more fluid samples for the presence, amount or identity of one or more analytes in the samples is provided, whereby the device comprises a substrate material and a means to at least partially elastically deform the substrate material,
- wherein the substrate material or defined areas on or at the substrate material are provided with at least one binding substance specific for at least one analyte,
- wherein the substrate material comprises an elastic porous material and one or more fluid samples, **characterized in that**
the substrate material is in an at least partially elastically deformed state by application of pressure applied by said means to at least partially elastically deform the substrate material and that the fluid within the substrate material has been laterally displaced in a direction essentially perpendicular to the direction that the pressure is applied to the substrate.

A device according to the present invention has the following advantages over the prior art:
- Due to the possibility of radial and/or lateral movement of fluid inside the substrate material, a better admixture of the fluid inside the substrate material can be achieved. Furthermore, since the whole amount of fluid may reach all binding substances for the analytes present in the fluid (which is not possible with a device e.g. as shown in the US 6, 849, 408 B2), the capture efficiency can be increased and therefore the sensitivity of the device may be furthermore enhanced. As a consequence precise detection of biomolecules can be performed with even a minute amount of analyte fluid.
- On the other hand it is possible to control the radial and/or lateral movement inside the substrate material through the amount of pressure that is applied onto the substrate material.

In the sense of the present invention, the term "elastic" especially describes a property of a material, that can be at least partially elastic deformed, i.e. the deformation is at least partially (or completely) reversible (gets its old shape, size, dimension back) if the pressure is released. Preferably a complete reversibility is achieved. The term "elastic" in the sense of the present invention especially means that also viscoelastic behaviour is included, i.e. the material showing some retardation in recovery after the stress is released (time effect).

In the sense of the present invention, the term "pressure" especially includes external stimuli (such as mechanical force, electric or magnetic force, light, air/gas pressure) on the substrate material that causes change of the properties of the substrate material e.g. change of thickness, (local) volume, size, porosity, or density.

In the sense of the present invention, the term "lateral and/or radial movement" means and/or includes especially
- a movement of fluid in a direction essentially perpendicular to that direction that pressure is applied on the substrate material, and/or
- a movement essentially parallel to the substrate surface, and/or
- that at least one component of the force/pressure, is perpendicular to the substrate surface, and/or
- that at least one component of the fluid movement is lateral, however the fluid movement may also include a transversal component.

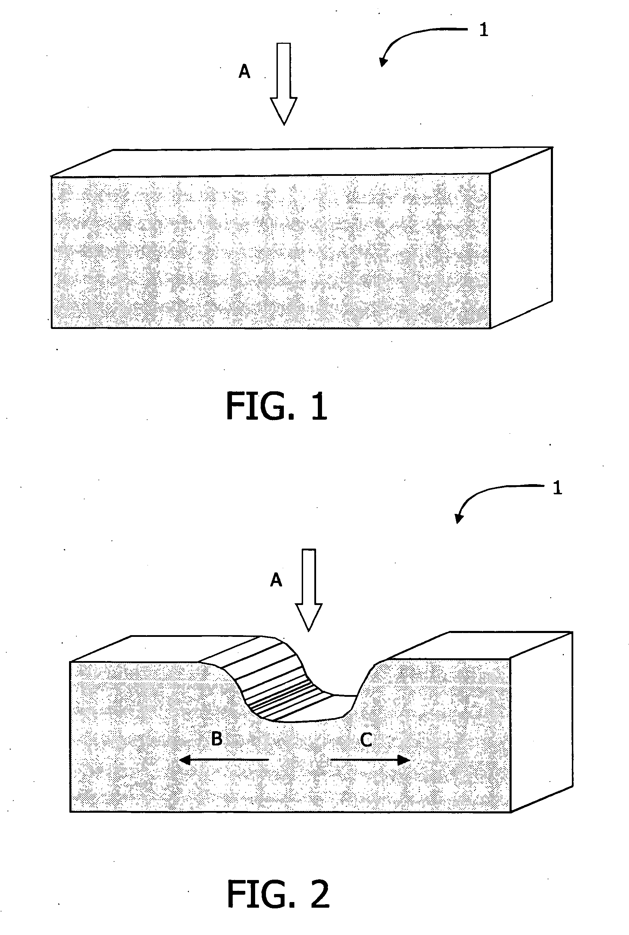

Moreover, the device for analyzing a fluid sample for an analyte is provided comprising a mechanism designed for moving a substrate material in a direction perpendicular to the access direction of the analyte. The term access direction is defined by a direction essentially normal to the substrate, as indicated by arrow A in Fig. 1. The wording direction perpendicular to the access direction is defined by all directions lying in the plane, which is essentially perpendicular to the access direction defined above. The mechanism may be designed in a variety of manners, for instance as described below. The mechanism may therefore comprise mechanical, electrical, magnetic or other appliances for exerting a force in order to move the substrate.

According to a preferred embodiment of the present invention, the substrate material is sensitive to pressure in that way that a radial lateral movement of fluid within the substrate material can be achieved by applying a force F on the substrate material from ≥1my and ≤50N. In this regard, the force F is the force that is applied by the pressure on the surface area. The pressure and the force F are connected by the equation p=F/A, i.e. force F divided by surface area A on which the force is applied. Preferably the force is ≥5mN and ≤5N, more preferred ≥10mN and ≤1N and most preferred ≥50man and ≤500mN.

In a preferred embodiment the surface area A on which the force is applied is only a part of the surface of the entire substrate material. Depending on the used means to apply a pressure A can vary between ≥100µm² up to ≤50mm², preferably ≥0.1mm² and ≤5mm² and is not limited to squared areas.

For person skilled in the art it is also evident, that always a counter force will be present, which could either be provided actively or taken over by any support structure.

According to a preferred embodiment of the present invention, the lateral displacement in the substrate material in dry condition (i.e. the substrate material is substantially dry before applying the fluid in the form of e.g. a droplet) without applying external pressure is so and ≤100µm. This has the advantage, that without applying external pressure the fluid will move lateral and/or radial inside the substrate material only to a certain extent. This allows a quicker analysis with a higher lateral resolution. Preferably the lateral displacement in the substrate material in dry condition without applying external pressure is ≤50µm and most preferred ≤10µm.

The term "lateral displacement" in the sense of the present invention means especially that the fluid (or some parts of the fluid) move inside the substrate material into a radial and/or lateral direction. For instance a droplet placed onto a porous, multidirectional substrate material in dry condition is sucked in and the spot size increases due to capillary force as known from a ink droplet placed on a sheet of paper.

According to a preferred embodiment of the present invention, the lateral displacement in the substrate material in wet condition without applying external pressure is ≥0 and ≤10µm, preferably ≤5µm.

According to a preferred embodiment of the present invention, the lateral displacement in the substrate material in wet condition with applying a pressure of 500kPa is ≥10µm and ≤5mm. This allows to control the lateral and/or radial movement of fluid inside the substrate material simply by applying pressure on the substrate material. Preferably, the lateral displacement in the substrate material in wet condition with applying a pressure of 500MPa is ≥50µm and ≤1mm, more preferred ≥100µm and ≤500µm.

In the sense of the present invention, the term "wet condition" especially includes the state at which the open, inner volume (i.e. all open pores) of the substrate material are substantially filled with a fluid. In the case of a substrate material with a pore size distribution of >100nm it includes especially the state at which at least all small (open) pores are filled and the fluid form a substantially connected (or linked) network inside the sponge like substrate material.

According to a preferred embodiment of the present invention, the lateral displacement in the substrate material in wet condition with applying a pressure of 10 MPa is ≥10µm and ≤5mm. This also allows to control the lateral and/or radial movement of fluid inside the substrate material simply by applying pressure on the substrate material. Preferably, the lateral displacement in the substrate material in wet condition with applying a pressure of 10 MPa is ≥50µm and ≤1mm, more preferred ≥100µm and ≤500µm.

According to a preferred embodiment of the present invention, the compression of the substrate material upon applying a pressure of 500kPa is ≥10µm and ≤500µm. By doing so, a good lateral and/or radial flow can be achieved. Preferably the compression of the substrate material is ≥50µm and ≤300µm.

According to a preferred embodiment of the present invention, the compression of the substrate material upon applying a pressure of 500kPa is ≥2% and ≤50% of its thickness.

According to a preferred embodiment of the present invention, the elastic modulus E of the substrate material the substrate material in wet condition is ≥0.5MPa and ≤5GPa. By doing so, a quick and reliable detection of the analytes can be achieved. Preferably the elastic modulus E of the substrate material is ≥1MPa and ≤1GPa and most preferred ≥5MPa and ≤100MPa.

According to a preferred embodiment of the present invention, the velocity of radial lateral movement of fluid within the substrate material in wet condition after applying a pressure is ≥1µm/s and ≤100m/s.

According to a preferred embodiment of the present invention, the substrate material is a porous material with an average pore size of ≥0,1 µm and ≤5 µm. Materials like these have proven themselves in practice.

According to a preferred embodiment of the present invention, the porosity (i.e. the ratio of the open, inner volume to the total volume) of the substrate material is ≥5% and ≤85%, more preferred ≥20% and ≤70% and most preferred ≥40% and ≤60%. In the sense of the present invention the term porosity does not include the inner closed volumes, as it can not be filled by liquid.

According to a preferred embodiment of the present invention, the substrate material is chosen from group comprising
- amorphous polymers, preferably from the group comprising PC (polycarbonate), PS (polystyrene), PMMA (polymethylmethacrylate), cellulose ester, cellulose nitrate, cellulose acetate, cellulose or mixtures thereof,
- semicrystalline polymers, preferably from the group comprising PA (polyamide= nylon materials), PTFE (polytrifluoroethylene), PE (polyethylene), PP (polypropylene), or mixtures thereof,
- rubbers, preferably from the group comprising PU (polyurethane), polyacrylates, silane based polymers such as PDMS (polydimethylsiloxane) or mixtures thereof,
- gels (= polymer networks with fluid solvent matrix), preferably from the group comprising agarose gel, polyacrylamide gel or mixtures thereof or mixtures thereof. These materials have shown to be suitable materials within the present invention.

The present disclosure furthermore relates to a method of making a substrate material according to the present invention, comprising the steps of
(a) printing at least one binding substance on the substrate material
(b) fixing the binding substance(s) on the substrate material
(c) drying the substrate material
and whereby the steps (b) and (c) may also be carried out in reverse order.

In the sense of the present invention, the term "printing" means especially that small fluid volumes like droplets comprising the binding substance in solution are accurately deposited onto predefined locations on the substrate material using techniques including micropipetting, ink jet printing and the like.

In the sense of the present invention, the term "fixing" means especially that a chemical reaction is initiated that establish a binding of the binding substance with the inner surface of the substrate material, initiated e.g. by UV-light irradiation.

Due to the fact, that the substrate material is on the one hand sensitive to pressure but on the other hand, according to a preferred embodiment of the present invention, the lateral displacement in dry condition is rather low, it is possible to simply print the binding substance(s) on the substrate material. In this state of the fabrication of the substrate material, the flow inside the substrate material should be as low as possible in order to control the position of the binding substance(s) on the substrate material. As a result small spots of a well defined size are obtained with a typical diameter in the range of 50-500µm. However, when applying the analyte, there should be a good flow inside the substrate material. This is achieved by providing a substrate material as described above.

The present invention furthermore relates to a method for analyzing one or more fluid samples for the presence, amount or identity of one or more analytes in the samples, comprising the steps of
(a) providing a substrate material as described above
(b) adding an amount of sample fluid to the substrate material, preferably to one or more defined areas on the substrate material
(c) applying a pressure on the substrate material as to achieve a radial lateral flow of fluid within the substrate material
(d) releasing the pressure on the substrate material as to allow the substrate material to return essentially to its shape prior to the application of pressure
(e) optionally repeating the two prior steps ad libitum
(f) optionally removing the sample fluid and/or washing the substrate material
(g) reading any signal generated in the substrate material and determining from said signal the presence, amount and/or identity of said one or more analytes in the sample.

By doing so a quick and reliable detection of analytes in the sample, even in minute amounts, is feasible. A person skilled in the art will recognize that at or between any of the steps (b) until (e) conditions suitable for binding the analyte to the binding substance (i.e. hybridization in case of DNA detection) are realized.

According to a preferred embodiment of the present invention, the method furthermore comprises a step (d2) which is between the steps (d) and (e) of (d2) reading any signal generated in the substrate material and determining from said signal the presence, amount and/or identity of said one or more analytes in the sample.

This embodiment allows to discriminate between high concentration of certain species in the analyte and lower concentration. In the first case one can detect a signal earlier, i.e. at step (d2), whereas in the second case, further "background signals", which might be present in the analyte need to be removed and the analysis is performed in step (g). By doing so, it is possible shorten the process even further, e.g. by stop repeating the steps for lateral fluid displacement if a dedicated signal is measured. (# remark: the term "pumping" was not introduced for this action)

According to a preferred embodiment of the present invention, the pressure in step (c) is applied for a time of ≥1s and ≤500s, preferably for a time of ≥5s and ≤50s.

According to a preferred embodiment of the present invention, the pressure in step (c) is between ≥100 kPa and ≤10 MPa and/or the pressure in step (c) is chosen as to apply a force F on the substrate material from ≥1mN and ≤50N.

According to a preferred embodiment of the present invention, the method furthermore comprises a step (c2), which is either before, between or after the steps (c) and (d) of:
(c2) applying one or more short pressure pulses (preferred duration is some milliseconds) causing a shock wave of the fluid in the substrate material.

According to this embodiment, the mixing inside the substrate material can be improved.

The present invention furthermore relates to a device comprising a substrate material as described above and/or adapted for conducting the method as described above. The device is equipped with means for applying pressure on the substrate material.

According to a preferred embodiment of the present invention, the means for applying pressure include
- mechanical means such as a rolling pin, a sphere, an indenter and/or
- air or gas pressure means, and/or
- electric means, such as electrodes, and/or
- magnetic means such as coils and/or magnetic particles.

According to a preferred embodiment of the present invention, the device comprises a system of electrodes in that way that pressure is applied to the substrate material by moving at least one electrode due to executing electrostatic force.

According to a preferred embodiment of the present invention, the electrostatic force is executed on the electrodes by a high frequency AC field. By doing so, unwanted electrolysis of an aqueous solution can be prevented.

According to a preferred embodiment of the present invention, additionally magnetic particles are embedded within the substrate material and application of pressure on the substrate material is executed by applying an external electromagnetic force.

According to a preferred embodiment of the present invention, the device comprises at least one rolling pin or a sphere to apply pressure on the substrate material.

A method and/or device according to the present invention may be of use in a broad variety of systems and/or applications, amongst them one or more of the following:
- biosensors used for molecular diagnostics
- rapid and sensitive detection of proteins and nucleic acids in complex biological mixtures such as e.g. blood or saliva
- high throughput screening devices for chemistry, pharmaceuticals or molecular biology
- testing devices e.g. for DNA or proteins e.g. in criminology, for on-site testing (in a hospital), for diagnostics in centralized laboratories or in scientific research
- tools for DNA or protein diagnostics for cardiology, infectious disease and oncology, food, and environmental diagnostics
- tools for combinatorial chemistry
- analysis devices.

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, features, characteristics and advantages of the object of the invention are disclosed in the dependent claims, the figures and the following description of the respective figures and examples, which -- in an exemplary fashionshow several preferred embodiments of a substrate material used in the method or device according to the invention as well as a device according to the invention.
Fig. 1 shows a very schematic cross-sectional view of a substrate material according to a first embodiment of the present invention;
Fig. 2 shows a very schematic cross-sectional view of the substrate material after application of pressure;
Fig. 3 shows a very schematic cross-sectional view of the substrate material after application of pressure at a place different than of Fig. 2;
Fig. 4 shows a very schematic top view of a device according to a second embodiment of the present invention;
Fig. 5 shows a schematic cross-section view of a device according to a third embodiment of the present invention applying mechanical pressure by a mangle configuration;
Fig. 6 shows schematic cross-section view of a device according to a fourth embodiment of the present invention applying mechanical pressure by a rolling pin configuration; and
Fig. 7 shows a very schematic cross-sectional view of a device according to a fifth embodiment of the present invention.

Fig. 1 shows a very schematic cross-sectional view of a substrate material 1 used in the present invention. In the detection sequence as described above, the substrate material is shown in the step after the sample, i.e. the analyte fluid, has been added to the substrate material but prior to the application of pressure on the substrate material. The application of pressure is executed in this embodiment approximately along arrow A.

Fig. 2 shows a very schematic cross-sectional view of the substrate material 1 after application of pressure along arrow A. This application then causes a radial lateral movement of the sample within the substrate material as indicated by the arrows B and C. It should be noticed that the movement of the substrate material is radial in that sense that the movement may as well also occur "outside the paper plane", which is not shown due to the perspective of Fig. 2.

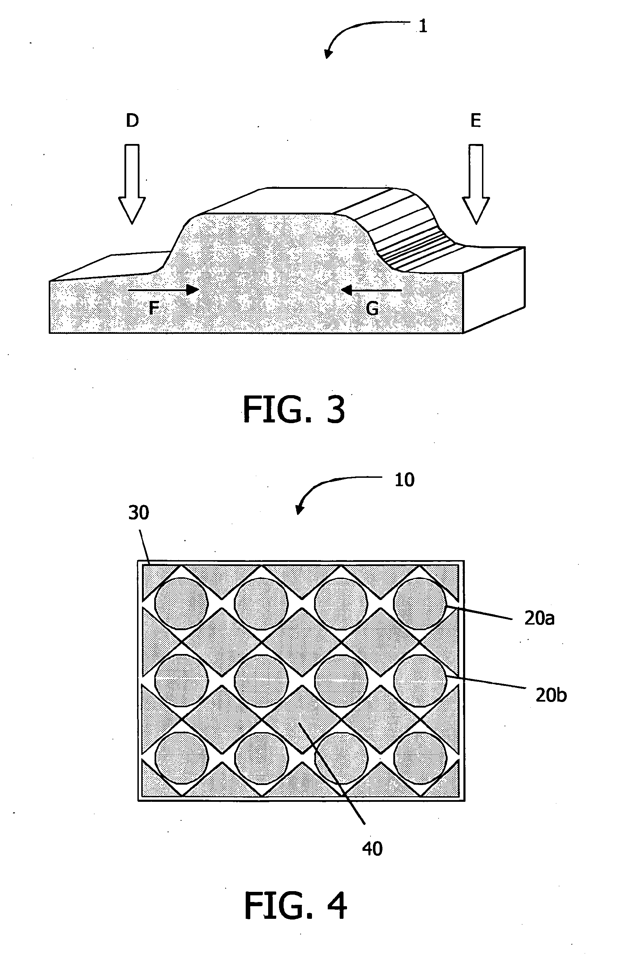

Fig. 3 shows a very schematic cross-sectional view of the substrate material after application of pressure at a place different than of Fig. 2. Here, at two places, i.e. in directions of the arrows D and E, pressure has been applied onto the substrate material, causing a lateral movement along the arrows F and G.

Fig. 4 shows a very schematic top view of a device 10 according to a second embodiment of the present invention. In the device 10, certain areas - two of which are exemplarily referred to as 20a and 20b of the substrate material have been provided with at least one binding substance specific for an analyte which is to be detected by the device. The nature of the binding substance itself is not part of the present invention and all techniques and methods known in the field may be used. However, usually the binding substance will e.g. be a single stranded DNA or RNA or a protein, an antibody for a certain protein or an enzyme or another biomolecule. It goes without saying that each of the areas 20a and 20b (and those not provided with numerals) is provided with a different binding substance or a different concentration of the same binding substance. For internal correction purpose some of the areas 20a and 20b might have the same binding substance.

The spaces between the areas (two of which have been referred to as 20a and 20b), which is exemplarily referred to as 30 or 40 is not provided with a binding substance.

When a sample is to be detected, the sample is brought on the device 10, preferably on the areas 20a, 20b and so on. After the sample has been applied to the substrate material, the sample is caused to move within the substrate material by executing pressure on the areas 20a, 20b and so on as described above. This will - ideally speaking - cause a uniform distribution of the sample within the substrate material. The substrate material is then released to return to its prior form.

In order to enhance the distribution of sample within the fluid, the execution of pressure may be repeated ad libitum. Usually the sample is then removed and the substrate material is washed.

In case an analyte which binds selectively e.g. to the binding substance of area 20a, the analyte will still be present after the removal of the sample and may then be detected. This can e.g. occur by fluorescence (in case the analyte has been marked with a fluorescent label) or by other suitable methods.

It should be noted that in this embodiment, the areas 20a, 20b, 30 and 40 differ only in the presence of a binding substrate. However, the device 10 may also be equipped with a mask or a pattern, which divides these areas from each other.

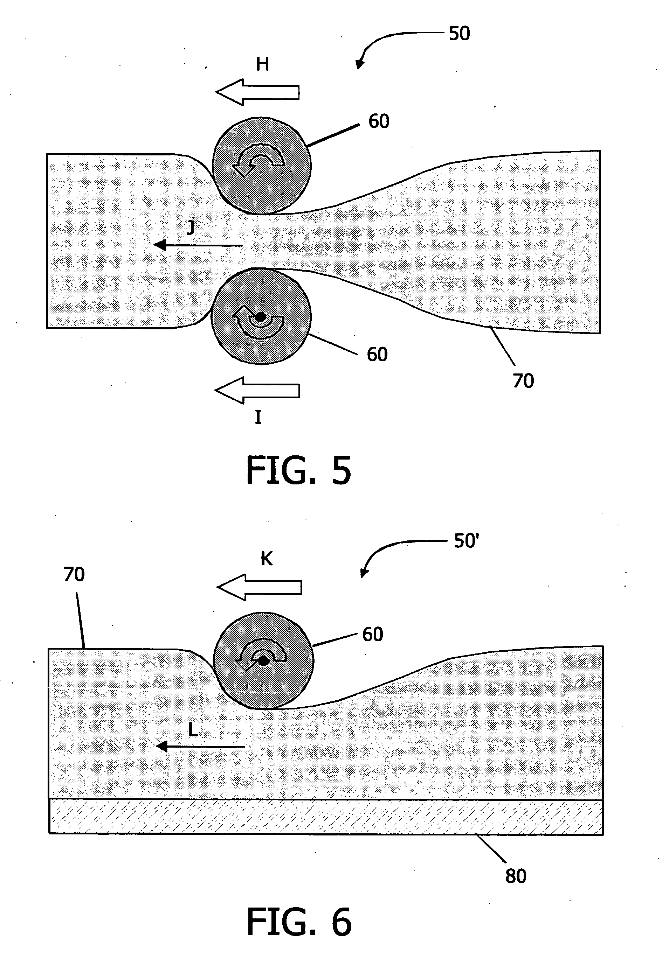

Fig. 5 shows a schematic cross-section view of a device 50 according to a third embodiment of the present invention applying mechanical pressure by a mangle configuration. In order to achieve this, two pins 60 (which are turning around in opposite directions as indicated with the arrows) move along the substrate material 70 somewhat in direction of the arrows H and I. By doing so, a very good lateral movement of fluid inside the substrate material 70 can be achieved in a "wave-like" form in the direction of the arrow J.

Fig. 6 shows schematic cross-section view of a device 50' according to a fourth embodiment of the present invention applying mechanical pressure by a rolling pin configuration. This device is quite similar to that shown in Fig. 5; it differs in that only one pin is provided (which moves in the same fashion as in Fig. 5 in direction of the arrow K) and there is a rigid "counterpart" 80 in form of a slab or a panel. By doing so, also a very good lateral movement of fluid inside the substrate material 70 in a "wave-like" form in direction of the arrow L can be achieved.

The rolling pins 60 in Figs. 5 and 6 may of course be also elliptical in form, thus causing a flow which is not uniform all the time. Further embodiments include pins which have indentations.

In this regard it should be noted that there are a variety of embodiments by which pressure can be applied on the substrate.

Another embodiment of the present invention (not shown in the figures) uses one or more spheres or spherical tips of an indenter device, which are indenting into the substrate material at a certain point. These spheres can be e.g. actuated simply by gravitation (i.e. "falling") but of course also a magnetic actuation can be used. In case an effect caused by gravitation force is used, the sphere can be realized with as heavy mass with a very tiny ball at the tip. If magnetic forces are used, a pole reversal may be used to lift the spheres again.

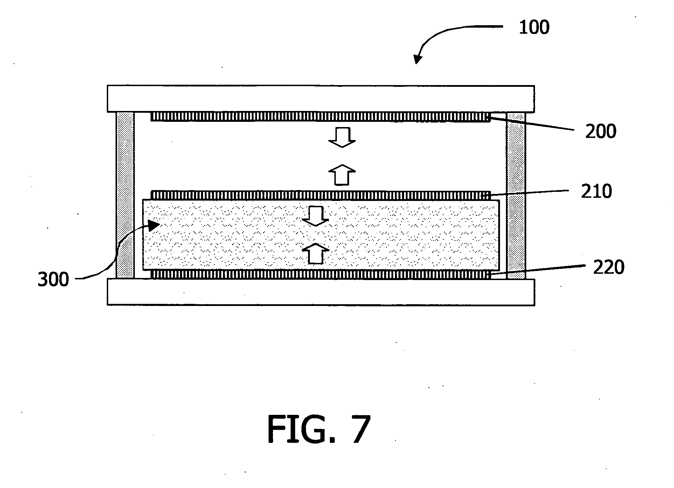

Fig. 7 shows a very schematic cross-sectional view of a device 100 according to a third embodiment of the present invention. In this device 100, the execution of pressure on the substrate material 300 is done via electrostatic force by using three electrodes 200, 210 and 220. Preferably the individually electrode segments of each electrode layer are connected in a matrix configuration (each layer has its own matrix) as known in the art.

The electrodes 200, 210 and 220 are provided in that way, that the electrode 210 may be either moved towards the electrode 200 or 220 (as indicated by the arrows). In order to execute pressure on the substrate material 300, the electrode 210 is caused to moved towards the electrode 200. In order to release the pressure, the electrode 210 is caused to move towards the electrode 220. By doing so, a controlled execution of pressure on the substrate material is easily possible which results in a quick and reliable detection of the sample.

In another preferred embodiment, the electrodes are realized as porous (permeable for the analyte fluid) layer on top (and bottom of or incorporated in) the substrate material (preferably segmented).

Contacting the substrate material in way that is known as peristaltic movement, realized e.g. by actuating neighboring electrodes (segments of the substrate material) in a predefined time sequential way will even further enhance the distribution of sample fluid. Therefore according to a further embodiment of the present invention, neighboring electrodes are provided with the substrate material in a suitable manner in order to achieve peristaltic movement.

According to a yet further embodiment of the present invention (not shown in the figs.) a magnetic system is used to apply pressure to the substrate material. This is preferably achieved by providing pairs of coils, preferably in form of spiral electrodes on both sides of the membrane. By applying an attracting magnetic field, pressure is applied on the substrate. In order to achieve this, it is preferred that either one coil is attracting the other coil or paramagnetic particles immobilized inside the porous membrane are attracted and thereby contracting the membrane. The repulsive force can according to a yet further embodiment of the present invention be provided by the elasticity of the polymer porous membrane - like in a sponge.

The particular combinations of elements and features in the above detailed embodiments are exemplary only. Accordingly, the foregoing description is by way of example only and is not intended as limiting. The scope of the invention is defined in the following claims. Furthermore, reference signs used in the description and claims do not limit the scope of the invention as claimed.

## Claims

1. A device comprising a substrate material and a means to at least partially elastically deform the substrate material,
wherein the substrate material or defined areas on or at the substrate material are provided with at least one binding substance specific for at least one analyte, wherein the substrate material comprises an elastic porous material and one or more fluid samples, **characterized in that**
the substrate material is in an at least partially elastically deformed state by application of pressure applied by said means to at least partially elastically deform the substrate material and that the fluid within the substrate material has been laterally displaced in a direction essentially perpendicular to the direction that the pressure is applied to the substrate.

2. The device according to claim 1, whereby the substrate material is chosen from the group comprising
- amorphous polymers, preferably from the group comprising PC (polycarbonate), PS (polystyrene), PMMA (polymethylmethacrylate), cellulose ester, cellulose nitrate, cellulose acetate, cellulose or mixtures thereof,
- semicrystalline polymers, preferably from the group comprising PA (polyamide= nylon materials), PTFE (polytrifluoroethylene), PE (polyethylene), PP (polypropylene), or mixtures thereof,
- rubbers, preferably from the group comprising PU (polyurethane), polyacrylates, silane based polymers such as PDMS (polydimethylsiloxane) or mixtures thereof,
- gels (= polymer networks with fluid solvent matrix), preferably from the group comprising agarose gel, polyacrylamide gel or mixtures thereof
or mixtures thereof.

3. A device according to claim 1, whereby the means to at least partially elastically deform the substrate material include
- mechanical means such as a rolling pin, a sphere, an indenter,
- electric means, such as electrodes and/or
- magnetic means such as coils and/or magnetic particles.

4. A method for analyzing one or more fluid samples for the presence, amount or identity of one or more analytes in the samples, comprising the steps of
(a) providing a substrate material, wherein the substrate material or defined areas on or at the substrate material are provided with at least one binding substance specific for at least one of said analytes, wherein the substrate material comprises an elastic porous material and wherein the substrate material can be at least partially elastically deformed by application of pressure
(b) adding an amount of sample fluid to the substrate material, preferably to one or more defined areas on the substrate material
(c) applying a pressure on the substrate material as to achieve a radial lateral flow of fluid within the substrate material
(d) releasing the pressure on the substrate material as to allow the substrate material to return essentially to its shape prior to the application of pressure
(e) optionally repeating the two prior steps ad libitum
(f) optionally removing the sample fluid and/or washing the substrate material
(g) reading any signal generated in the substrate material and determining from said signal
the presence, amount and/or identity of said one or more analytes in the sample.

5. A system adapted to conduct the method of claim 4 and incorporating a device according to claim 1 to 3 and being used in one or more of the following applications:
- biosensors used for molecular diagnostics
- rapid and sensitive detection of proteins and nucleic acids in complex biological mixtures such as e.g. blood or saliva
- high throughput screening devices for chemistry, pharmaceuticals or molecular biology
- testing devices e.g. for DNA or proteins e.g. in criminology, for on-site testing (in a hospital), for diagnostics in centralized laboratories or in scientific research
- tools for DNA or protein diagnostics for cardiology, infectious disease and oncology, food, and environmental diagnostics
- tools for combinatorial chemistry
- analysis devices.

## Patentansprüche

1. Vorrichtung mit einem Substratmaterial und einem Mittel, um das Substratmaterial zumindest teilweise elastisch zu verformen,
wobei das Substratmaterial oder definierte Bereiche auf oder bei dem Substratmaterial mit mindestens einer bindenden Substanz versehen sind, die für mindestens einen Analyten spezifisch ist, wobei das Substratmaterial ein elastisches poröses Material und eine oder mehrere Fluidproben umfasst, **dadurch gekennzeichnet, dass**
sich das Substratmaterial in einem zumindest teilweise elastisch verformbaren Zustand befindet, indem durch das genannte Mittel Druck ausgeübt wird, um das Substratmaterial zumindest teilweise elastisch zu verformen, und dass das Fluid in dem Substratmaterial seitlich in eine Richtung verdrängt wurde, die im Wesentlichen senkrecht zu der Richtung verläuft, in der der Druck auf das Substrat ausgeübt wurde.

2. Vorrichtung nach Anspruch 1, wobei das Substratmaterial aus der Gruppe, die Folgendes umfasst:
- amorphe Polymere, vorzugsweise aus der Gruppe, die PC (Polycarbonat), PS (Polystyrol), PMMA (Polymethylmethacrylat), Zelluloseester, Zellulosenitrat, Zelluloseacetat, Zellulose oder Gemische daraus umfasst,
- semikristalline Polymere, vorzugsweise aus der Gruppe, die PA (Polyamid = Nylon-Materialien), PTFE (Polytrifluoroethylen), PE (Polyethylen), PP (Polypropylen) oder Gemische daraus umfasst,
- Gummi, vorzugsweise aus der Gruppe, die PU (Polyurethan), Polyacrylate, silanbasierenden Polymere wie PDMS (Polydimethylsiloxan) oder Gemische daraus umfasst,
- Gele (= Polymernetzwerke mit fluider Lösungsmittelmatrix), vorzugsweise aus der Gruppe, die Agarose-Gel, Polyacrylamid-Gel oder Gemische daraus umfasst,
oder aus Gemischen daraus gewählt wird.

3. Vorrichtung nach Anspruch 1, wobei die Mittel zum zumindest teilweise elastischen Verformen des Substratmaterials Folgendes umfassen:
- mechanische Mittel wie eine Walzenstift, eine Kugel, einen Stempel,
- elektrische Mittel wie Elektroden und/oder
- magnetische Mittel wie Spulen und/oder magnetische Partikel.

4. Verfahren zum Analysieren von einer oder mehreren Fluidproben auf die Anwesenheit, die Menge und die Identität von einem oder mehreren Analyten in den Proben, wobei das Verfahren aus den folgenden Schritten besteht:
a) Schaffen eines Substratmaterials, wobei das Substratmaterial oder definierte Bereiche auf oder bei dem Substratmaterial mit mindestens einer bindenden Substanz versehen sind, die für mindestens einen der genannten Analyten spezifisch ist, wobei das Substratmaterial ein elastisches poröses Material umfasst und wobei das Substratmaterial durch das Ausüben von Druck zumindest teilweise elastisch verformt werden kann,
b) Zugeben einer Probenfluidmenge zu dem Substratmaterial, vorzugsweise zu einem oder mehreren definierten Bereichen auf dem Substratmaterial
c) Ausüben eines Drucks auf das Substratmaterial, um einen radialen lateralen Fluss des Fluids in dem Substratmaterial zu erreichen
d) Entlasten des Substratmaterials von dem Druck, damit das Substratmaterial im Wesentlichen in seine Form vor dem Ausüben des Drucks zurückkehren kann
e) optional beliebiges Wiederholen der beiden vorhergehenden Schritte
f) optional Entfernen des Probenfluids und/oder Waschen des Substratmaterials
g) Lesen jedes in dem Substratmaterial erzeugten Signals und anhand des genannten Signals die Anwesenheit, die Menge und/oder die Identität des genannten einen oder
der genannten mehreren Analyten in der Probe bestimmen.

5. System, das vorgesehen ist, um das Verfahren nach Anspruch 4 durchzuführen, eine Vorrichtung nach Anspruch 1 bis 3 enthält und in einer oder mehreren der folgenden Anwendungen verwendet wird:
- Biosensoren für die Molekulardiagnose,
- schnelle und empfindliche Detektion von Proteinen und Nukleinsäuren in komplexen biologischen Gemischen wie z.B. Blut oder Speichel,
- Screening-Vorrichtungen mit hohem Durchsatz für Chemie, Pharmazie oder Molekularbiologie,
- Testvorrichtungen z. B. für DNA oder Proteine in z. B. der Kriminologie, für die Prüfung vor Ort (in einem Krankenhaus), für die Diagnose in zentralisierten Laboratorien oder in der wissenschaftlichen Forschung,
- Werkzeuge für die DNA- oder Proteindiagnose in den Bereichen Kardiologie, Infektionskrankheiten und Onkologie, Nahrungsmittel, und Umgebungsdiagnose
- Werkzeuge für die kombinatorische Chemie,
- Analysevorrichtungen.

## Revendications

1. Dispositif comprenant un matériau de substrat et des moyens pour déformer au moins partiellement élastiquement le matériau de substrat,
dans lequel le matériau de substrat ou les zones définies sur ou au niveau du matériau de substrat est (sont) doté(es) d'au moins une substance liante spécifique pour au moins une substance à analyser, dans lequel le matériau de substrat comprend un matériau poreux élastique et un ou plusieurs échantillons de fluide, **caractérisé en ce que**
le matériau de substrat se trouve dans un état au moins partiellement élastiquement déformé à cause de l'application de pression appliquée par lesdits moyens pour déformer au moins partiellement élastiquement le matériau de substrat et **en ce que** le fluide à l'intérieur du matériau de substrat a été déplacé latéralement dans une direction essentiellement perpendiculaire à la direction dans laquelle la pression est appliquée au substrat.

2. Dispositif selon la revendication 1, moyennant quoi le matériau de substrat est choisi dans le groupe comprenant
- des polymères amorphes, de préférence dans le groupe comprenant PC (polycarbonate), PS (polystyrène), PMMA (polyméthylméthacrylate), ester de cellulose, nitrate de cellulose, acétate de cellulose, cellulose ou mélanges de ceux-ci,
- des polymères semi-cristallins, de préférence du groupe comprenant PA (polyamide = matériaux de Nylon), PTFE (polytrifluoroéthylène), PE (polyéthylène), PP (polypropylène) ou mélanges de ceux-ci,
- des caoutchoucs, de préférence du groupe comprenant PU (polyuréthane), polyacrylates, polymères à base de silane tels que PDMS (polydiméthylsiloxane) ou mélanges de ceux-ci,
- des gels (= réseaux polymères avec matrice de solvant fluide), de préférence du groupe comprenant gel d'agarose, gel de polyacrylamide ou mélanges de ceux-ci ou mélanges de ceux-ci.

3. Dispositif selon la revendication 1, dans lequel les moyens pour déformer au moins partiellement élastiquement le matériau de substrat comprennent
- des moyens mécaniques tels qu'un axe de roulement, une sphère, un indenteur,
- des moyens électriques, tels que des électrodes et/ou
- des moyens magnétiques tels que des bobines et/ou particules magnétiques.

4. Procédé pour analyser un ou plusieurs échantillons de fluide afin de vérifier la présence, la quantité ou l'identité d'une ou plusieurs substances à analyser dans les échantillons, comprenant les étapes consistant à
(a) fournir un matériau de substrat, dans lequel le matériau de substrat ou les zones définies sur ou au niveau du matériau de substrat sont dotées d'au moins une substance liante spécifique pour au moins l'une desdites substances à analyser, dans lequel le matériau de substrat comprend un matériau poreux élastique et dans lequel le matériau de substrat peut être au moins partiellement déformé élastiquement par application de pression
(b) ajouter une quantité de fluide d'échantillonnage au matériau de substrat, de préférence sur une ou plusieurs zone(s) définie(s) sur le matériau de substrat
(c) appliquer une pression sur le matériau de substrat afin d'obtenir un écoulement latéral radial de fluide à l'intérieur du matériau de substrat
(d) relâcher la pression sur le matériau de substrat afin de permettre au matériau de substrat de reprendre essentiellement la forme qu'il avait avant l'application de pression
(e) répéter en option les deux étapes antérieures ad libitum
(f) retirer en option le fluide d'échantillonnage et/ou laver le matériau de substrat
(g) lire n'importe quel signal généré dans le matériau de substrat et déterminer à partir dudit signal la présence, la quantité et/ou l'identité desdites une ou plusieurs
substances à analyser dans l'échantillon.

5. Système adapté pour effectuer le procédé selon la revendication 4 et intégrant un dispositif selon les revendications 1 à 3 et étant utilisé dans une ou plusieurs des applications suivantes :
- biocapteurs utilisés pour les diagnostics moléculaires
- détection rapide et sensible de protéines et d'acides nucléiques dans des mélanges biologiques complexes tels que par exemple, le sang ou la salive
- dispositifs de criblage à haut rendement pour la chimie, les produits pharmaceutiques ou la biologie moléculaire
- dispositifs d'essai pour l'ADN et les protéines, en criminologie par exemple, pour l'essai sur site (dans un hôpital), pour des diagnostics dans des laboratoires centralisés ou dans la recherche scientifique
- outils pour des diagnostics d'ADN ou de protéines pour des diagnostics cardiologiques, de maladies infectieuses et oncologiques, alimentaires et environnementaux
- outils pour la chimie combinatoire.
- dispositifs d'analyse.
